# EUROPEAN PATENT APPLICATION

(11) **EP 4 534 111 A1**
(43) Date of publication of application: **09.04.2025**
(21) Application number: 23815357.1
(22) Date of filing: 02.06.2023
(51) Int. Cl.: A61L 2/03

(54) **DEVICE, SYSTEM AND ACTIVATION METHOD FOR INTRAOPERATIVELY DISINFECTING BONE PROSTHESES USING BIOELECTRIC EFFECT**

(30) Priority: 02.06.2022 ES 202230480
(71) Applicant: Universidad De Malaga, 29071 Málaga (ES); Servicio Andaluz de Salud, 41071 Sevilla (ES)
(72) Inventor: TAMIMI MARIÑO, Iskandar, 41071 Sevilla (ES); GOMEZ DE GABRIEL, Jesús Manuel, 29071 Málaga (ES); URDIALES GARCÍA, Amalia Cristina, 29071 Málaga (ES); TAMIMI MARIÑO, Faleh, Montreal, Québec H3A 0G4 (CA); GASCA, María, 41071 Sevilla (ES)
(74) Representative: TRBL Intellectual Property
(86) International application number: PCT/ES2023/070371
(87) International publication number: WO 2023/233063

(57) **Abstract**

The present invention relates to a device for intraoperatively disinfecting bone prostheses (7) using bioelectric effect. Advantageously, the device comprises an array (1) that can be adapted to the shape of a bone prosthesis (7), wherein said array (1) is provided with an arrangement of electrodes (2) designed to connect to a programmable controller (11) for supplying current, designed to perform a bioelectric treatment on the prothesis (7). The invention also relates to a disinfection system comprising the device connected to a programmable controller (11) for supplying current, and to a method for activating same.

## Description

### FIELD OF THE INVENTION

The present invention is comprised in the medical sector, and more specifically in the surgical field. Its application focuses mainly on preventing and intraoperatively eliminating infections in knee prostheses, but without limitation to another type of prostheses or applications, by means of the application of low-power electric currents.

### BACKGROUND OF THE INVENTION

Bacterial biofilm formation in bone implants such as, for example, prosthetic knee implants, is a post-surgical complication that leads to the chronification of infections, as well as the protection of bacterial colonies against antibiotics. Biofilms are mainly generated by the adhesion of microorganisms to the metal surfaces of the prosthesis, mainly by the action of Van del Waals forces, electrostatic forces, and acid-base interaction. The surface on which biofilms are formed also contains enzymes that trap antimicrobial agents, which decreases the penetration of antibiotics, reducing their effectiveness.

Treatment of prosthetic bone infections is complex, time-consuming, and costly. Traditionally, it consists of washing and replacing mobile components in recent implant infections (between three and six weeks after surgery) using antibiotic-impregnated cements and intraoperative irrigation. However, biofilm can remain in a state of hibernation for prolonged periods of time, making it significantly more difficult to detect using traditional techniques. After the mentioned initial period following surgery, treatment of infections generally requires a complete replacement of the implant in a single surgery or, alternatively, a first replacement with a temporary component which, after a minimum period of six weeks, is replaced in a second intervention by a definitive implant. The first method has a success rate between 50 and 55%, while the success rate of the second method goes up to 70-90%. In spite of this, in either case these methods have an enormous physical and psychological cost for the patient, in addition to a high economic cost, which in the case of knee prostheses is estimated at about twenty billion dollars worldwide, corresponding to infection in 1%-2% of cases, which accounts for about three hundred thousand infected prostheses per year. Additionally, it should be noted that a considerable number of infections are not resolved with these methods, which can lead to amputation or even death of the patient. The most recent studies indicate that five-year mortality after prosthetic infection reaches 25.9% compared to 12.0% in patients without infection.

As a complement to conventional treatments, different studies published in recent years point to the use of the so-called "bioelectric effect", the effectiveness of which has been confirmed in colonies of *Escherichia coli* in saline solution, *Staphylococcus aureus* in agar, skin flora, *Proteus* and *Klebsiella pneumoniae* in synthetic urine and *Escherichia coli, Staphylococcus aureus* and *Bacillus subtilis* in water. The cause of this bactericidal effect apparently lies in the substances produced by electrolysis, cytoplasmic membrane rupture or decreased bacterial respiration. However, the experiments designed to date along these lines have only been conducted *in vitro* and, furthermore, their application requires very long application periods of up to sixteen hours in some cases. Some recent studies propose mixed treatments based on the combination of the bioelectric effect with antibiotics to improve the effectiveness of the latter, although, again, they are only designed for *in vitro* tests at present.

Therefore, there is a need in the present technical field to develop improved devices and methods for disinfecting prostheses based on bioelectric effect, which allow their application to *in vivo* techniques in a shorter time compared to the known methods of the state of the art. The present invention seeks to fulfil said need by means of a novel device, system and method for intraoperatively disinfecting bone prostheses, the preferred application of which is the treatment of knee prostheses, but without limitation to the use thereof in another type of implants.

### BRIEF DESCRIPTION OF THE INVENTION

As described above, a first object of the invention relates to a device for intraoperatively disinfecting bone prostheses, which allows both weakening biofilms and eliminating bacterial infections using bioelectric effect. The application of said device focuses mainly on knee prosthesis implantation surgery, by means of the selective application of low-intensity currents on the surface of the prosthesis, optionally in combination with the subsequent application of antibiotics, which would improve the effectiveness of the latter as a result of prior weakening or elimination of the biofilm.

More specifically, the device of the invention comprises an array that can be adapted to the shape of a bone prosthesis, wherein said array is provided with an arrangement of electrodes adapted to be connect to a programmable controller for supplying current, and configured to perform a bioelectric treatment on the prosthesis.

Both the array and the other elements of the device of the invention are advantageously designed for using the device intraoperatively on the prosthesis, where it can be removed from the prosthesis after performing the bioelectric treatment. Therefore, the device represents an improved solution with respect to other known disinfection devices based, for example, on implantable elements that use electrodes made up of two conductive surfaces (an anode and a cathode) and are applied on medical implants and catheters. Unlike the approach of the present invention, these devices cannot be placed on metal surfaces, since they would act as transmitters between the two electrodes, whereby the main functionality of the devices would be lost.

Therefore, the present invention is proposed as a device preferably adapted with separating means adapted to be fixed between the prosthesis and the array of electrodes, maintaining a minimum distance between said array and the metal elements of the prosthesis or implant. In addition to the above, the device is reusable in nature, allowing it to be removed from the prosthesis or implant before the surgery ends, obtaining less invasive disinfecting means.

The fact that the device of the invention does not have to be incorporated as part of the prosthesis or implant itself is another significant advantage, related to the fact that, in general, prostheses (and, in a particularly relevant manner, knee prostheses) must have clear and smooth surfaces since they are subjected to very high mechanical loads ranging between 40 N/cm² and 150 N/cm². These cyclic pressures would destroy any implant placed on the surface thereof that was not specifically designed to withstand mechanical loads of those mentioned. Therefore, the intraoperative (non-implantable) and reusable nature of the device of the invention eliminates electrochemical wear, abrasion, and mechanical erosion of the prosthesis, compared to known implantable devices.

Finally, and as an additional advantage, the device of the invention uses the same prosthesis as part of the set of electrodes (acting as an anode or a cathode that is common to the rest of the terminals, depending on which the excitation signal is). As a result of the above, current is transmitted through the outer surface of the prothesis, which is precisely where a greater biofilm weakening effect is to be generated. This concept is also a differentiating concept with respect to the state of the art and is generally applicable to practically all existing metal implants. Furthermore, as a result of its operating principle, the time required to eradicate bacterial colonies can be shortened considerably. In this sense, although known devices require several days of treatment to have minimum effectiveness, the present invention can obtain satisfactory disinfection results within a few minutes. Accordingly, this allows the invention to be advantageously applied during surgery, unlike devices of the state of the art.

In a preferred embodiment of the invention, the array is made of a flexible material, on which there is arranged a plurality of conductive tracks connected to a plurality of corresponding electrodes.

In another preferred embodiment of the invention, the array comprises a central portion that can be adapted to the front surface of a knee prosthesis, and two side portions that can be adapted to the corresponding side surfaces of said prosthesis.

In another preferred embodiment of the invention, the array comprises one or more reference electrodes adapted for direct contact with the bone prosthesis.

In another preferred embodiment of the invention, the array comprises an appendage, by way of a flat cable, ending in a connector adapted to connect the device to a programmable controller for supplying current.

In another preferred embodiment of the invention, the array comprises one or more openings adapted to favor serum circulation during the operation of the device.

In another preferred embodiment of the invention, the device further comprises one or more supports for fixing the array to the prosthesis. More preferably, said supports are adapted such that, when the device is positioned on the prosthesis, the electrodes are arranged respecting a minimum distance with respect to said prosthesis. Even more preferably, the device comprises two supports, wherein:
- a first support comprises a grid, by way of a separator, between the array and the prosthesis, designed to limit or prevent direct electrical contact between the prosthesis and the electrodes; and wherein
- a second support is arranged over the array, by way of a casing, structurally reinforcing the shape adopted by the array and the first separating support.

In another preferred embodiment of the invention, the first separating support is made with flexible materials, and/or the second structural support is made with a rigid material.

In another preferred embodiment of the invention, the second structural support comprises one or more holes adapted to favor serum circulation during the operation of the device.

In another preferred embodiment of the invention, the second structural support comprises one or more positioning elements adapted to position one or more reference electrodes of the array in contact with the bone prosthesis.

As mentioned, the novel design of the device of the invention allows activating the electrodes in a coordinated and selective manner, generating currents in selected areas of the surface of the prosthesis for a controlled time, thereby eliminating the biofilm by attacking possible bacterial colonies and improving the effect of the antibiotic in the clean area. Likewise, the device poses multiple advantages with respect to known prosthesis disinfection systems including: i) the device allows ergonomic and simple application on the surface of the prothesis when it is already adhered to the knee; ii) as a result of its design, the current application time can be in accordance with the planned intervention time; iii) the current intensity allows application in a completely safe range so as not to damage living tissue; iv) the device is readily portable, compact and safe, suitable for an operating room; v) the part of the device in contact with the patient can be disposed of and replaced.

A second object of the invention relates to a system for intraoperatively disinfecting bone prostheses using bioelectric effect, comprising a device for disinfecting according to any of the embodiments described herein, and wherein said device is connected to a programmable controller for supplying current, connected to the device and adapted to perform a bioelectric treatment on the prosthesis through the electrodes.

In a preferred embodiment of the invention, the device and the controller are connected in a modular manner through a connector that can be coupled and uncoupled.

In another preferred embodiment of the invention, the controller comprises a signal generator connected to programmable waveform-generating means, said generator and the programmable means being adapted to conduct said waveforms, in a coordinated or selective manner, to the electrodes of the device.

In another preferred embodiment of the invention, the controller comprises one or more amplifiers and a relay block adapted to select the electrodes of the array to which the power waveform signal is sent, and those which are disconnected. More preferably, one or more reference electrodes are connected to the amplifiers through a grounding line. Furthermore, even more preferably, the controller comprises a set of current sensors connected to the relay block and to the programmable current control means.

In another preferred embodiment of the invention, the programmable current control means are adapted with hardware and/or software means to calculate the energy supplied to the electrodes and to deactivate them when they have reached a programmed maximum energy value.

In another preferred embodiment of the invention, the controller comprises a user interface connected to the programmable current control means adapted to display and program the operating parameters of the system, as well as to monitor and control the operating condition thereof.

A third object of the invention relates to an activation method for activating a system according to any of the embodiments described herein, which comprises operating the controller for applying current to activate at least one of the electrodes arranged in the array of the device for disinfecting.

In a preferred embodiment of the invention, the electrodes are activated by means of at least one of the following modes:
- sequential, wherein there is only one active electrode at a time;
- parallel, wherein several or all of the electrodes are activated at the same time.

Finally, a fourth object of the invention relates to a method for intraoperatively disinfecting bone prostheses using bioelectric effect, which comprises using a system according to any of the embodiments described herein and performing the following steps:
- the prosthesis is exposed;
- the array of electrodes is fixed to the prosthesis;
- one or more regions to be disinfected of the prosthesis are bathed in saline solution;
- the array is connected to the controller;
- a current sequence in the electrodes is activated with the controller, causing it to pass to the prosthesis through the saline solution and/or by direct contact.

In a preferred embodiment of the invention, the electrode activation sequence is maintained for a time comprised between 10 and 30 minutes, and more preferably between 15 and 25 minutes.

The current application time can be reduced if the current sensors detect that the desired energy has been reached before the expected time. Once said time has elapsed, the system generates a warning and the device is removed from the prosthesis at that moment. The prosthesis can be washed with a sponge and disinfectant, and irrigated with physiological saline solution. Finally, the surgical wound is closed by layers.

### DESCRIPTION OF THE DRAWINGS

Figure 1 shows a device for disinfecting of the invention according to a preferred embodiment thereof based on an array of electrodes.
Figure 2 shows an exploded view of the device of the invention according to a preferred embodiment thereof based on an array of electrodes and two support elements that act as a spacer and structural reinforcement, respectively.
Figures 3-4 show perspective views of the device of Figure 2, with its elements assembled and fixed to a bone prosthesis.
Figure 5 shows the system for disinfecting of the invention, according to a preferred embodiment thereof.

### Reference numbers used in the drawings:

- (1): Array that can be adapted to the shape of a bone prosthesis.
- (1'): Central portion of the array.
- (1"): Side portions of the array.
- (2): Electrodes.
- (3): Reference electrodes.
- (4): Appendage.
- (5): Connector.
- (6): Openings of the array.
- (7): Prosthesis.
- (8): First separating support for separating the array from the prosthesis.
- (9): Second structural support of the array.
- (9'): Holes of the second structural support.
- (10): Positioning elements.
- (11): Controller for applying current.
- (12): Signal generator
- (13): Programmable waveform-generating means.
- (14): Amplifier.
- (15): Relay.
- (16): Grounding line.
- (17): Current sensors.
- (18): User interface.

### DETAILED DESCRIPTION OF THE INVENTION

As described in the preceding sections, the present invention relates, in a first object thereof, to an electronic device which allow applying bioelectric effect to a bone prosthesis, and preferably to a knee prosthesis, during a surgical intervention to destroy or weaken a biofilm surface formed on said prosthesis and to prevent possible bacterial infections, by itself or by means of its synergistic effect with antibiotic treatment.

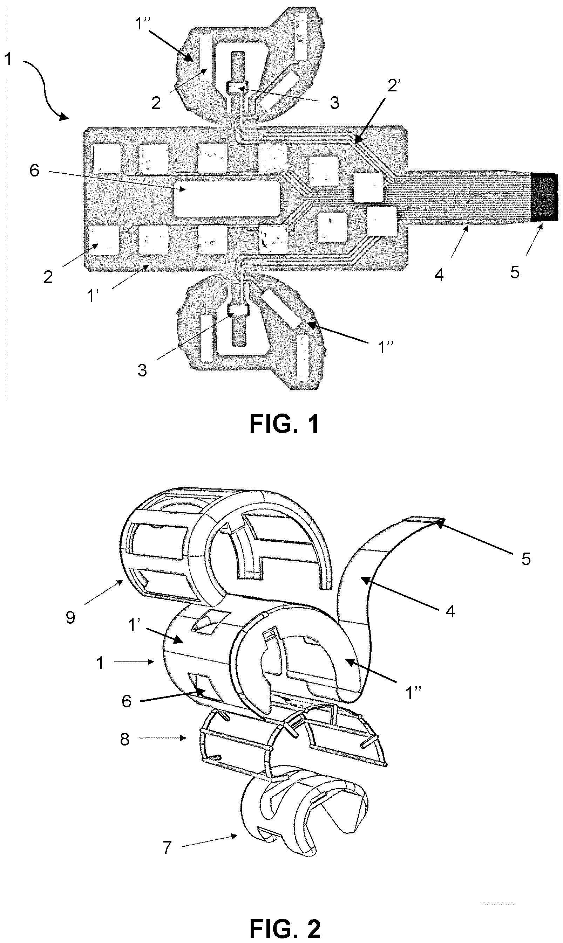

To that end, and as shown in Figure 1 herein, said electronic device comprises an array (1) that can be adapted to the shape of a bone prosthesis, wherein said array (1) is provided with an arrangement of electrodes (2), through which a current can be applied in a programmed manner in order to perform a bioelectric treatment on said prosthesis. Preferably, the array (1) is made of a flexible material, for example, an FPCB (Flexible Printed Circuit Board) type material, on which there is arranged a plurality of conductive tracks (2') (made of silver or another similar conductive material, for example), connected to a plurality of corresponding electrodes (2). The main purpose of the array (1) is to provide a surface that can be adapted to the shape of the bone prosthesis, either by means of a rigid or semirigid material that already has said shape or, preferably, by means of a flexible material that can be adapted to same (like the case of FPCBs, manufactured mainly with flexible plastic materials). By way of example, the design of the array (1) shown in Figure 1 can be adapted to the shape of a knee prosthesis and comprises a central portion (1') that can be adapted to the front surface of said prosthesis and two side portions (1") that can be adapted to the corresponding side surfaces thereof.

In a preferred embodiment of the invention, also as depicted in Figure 1, the array (1) comprises one or more reference electrodes (3), preferably arranged in the side portions (1") of the array (1) and adapted for direct contact with the bone prosthesis. More preferably, the array (1) comprises an appendage (4), by way of a flat cable, ending in a direct-insertion connector (5), which keeps the electrical connections of the array (1) away from the sterile area of the prosthesis. Finally, in a preferred embodiment of the invention the array (1) may also comprise one or more openings (6) which advantageously allow serum circulation during the operation of the device.

The design of the array (1) of electrodes (2) described above can be manufactured with low-cost materials, allowing it to be of single use, which is suitable for health and surgical application. More specifically, FPCBs can withstand elevated temperatures, as well as sterilization processes by means of chemical substances, so, where appropriate, they could also be reused if desired. Due to their flexible nature, the use of FPCBs also allows adapting the array (1) to diverse types of curved surfaces, where different bending lines which allow coupling the device to the desired prosthesis in a quick and simple manner during application can be defined during manufacture.

In another preferred embodiment of the invention shown in Figures 2-4, one or more supports (8, 9) can further be used to fix the array (1) to the prosthesis (7), securing the positioning of said array (1) and its electrodes (2), as well as the contact of the reference electrodes (3), if they are used, in relation to the metal surfaces of the prosthesis (7), in a quick and simple manner. In said embodiment, the supports (8, 9) are adapted such that, when the device is positioned on the prosthesis (7), the electrodes (2) are arranged respecting a minimum distance with respect to the prosthesis (except, where appropriate, one or more reference electrodes (3) that will be in direct contact with the prosthesis (7)). When using the device, and once the array (1) is positioned on the prosthesis (7), the assembly is submerged in a saline solution, such that each electrode (2) allows generating, in a controlled manner, an electric current that is conducted to a different location on the surface of the prosthesis (7). This approach allows an improved coverage of the irregular surfaces thereof, as well as a more precise current application. Furthermore, as will be described in the following sections, regulation of the activation sequence of the different electrodes (2) allows ensuring that the current is homogenously distributed throughout the entire surface of the prosthesis (7). Likewise, the number of electrodes (2) determines the resolution with which the energy applied to the surface of the prosthesis (7) can be metered.

In the specific embodiment illustrated by Figures 2-4, it can be seen how the array (1) of electrodes (2) of the device comprises two supports (8, 9), wherein a first support (8) comprises a grid, by way of a separator, ensuring that there is no direct electrical contact between the prosthesis (7) and the electrodes (2), and that the array (1) adapts to the curvature of the prosthesis (7) (in the example shown, a femoral knee prosthesis (7), although in other embodiments the device can be adapted to other types of prosthetic implants). As a result of this configuration, the array (1) of electrodes (2) is bent and folded over the first separating support (8). Preferably, said first support (8) is made with flexible materials, for example plastic materials, to also make it better adapt to the shape of the prosthesis (7).

In a complementary manner, the device shown in Figures 2-4 comprises a preferably rigid or semirigid second support (9) arranged in the top portion thereof, for example, by way of a casing, structurally reinforcing the shape adopted by the array (1) of electrodes (2) and the first separating support (8), holding the assembly formed by the elements of the device. In different embodiments of the invention, the second structural support (9) may optionally comprise one or more holes (9') adapted to enable visually controlling the suitable positioning of the array (1) on the prothesis (7), as well as to favor suitable serum circulation during the operation of the device, like the openings (6). More preferably, the second structural support (9) may comprise one or more positioning elements (10) adapted to position the reference electrodes (3) (if they are used) of the array (1) in contact with the prothesis (7).

In addition to the device described in the preceding paragraphs, a second object of the invention relates to a system for intraoperatively disinfecting bone prostheses using bioelectric effect which comprises, in addition to said device, a controller (11) for supplying current, which is connected to the device through the connector (5). In said system, the device and the controller (11) are adapted to be coupled in a modular manner, such that a controller (11) can be readily connected to and disconnected from any array (1) of electrodes (2). As mentioned, this further favors the use of disposable arrays (1) that can be replaced with other arrays to perform a new disinfection method.

Therefore, the controller (11) is the element of the system which allows measuring, managing and operating the independent activation of the electrodes (2) in order to control the total energy supplied, both individually and as a whole, to the different regions of the prothesis (7), in order to promote homogenous current distribution throughout the entire surface thereof.

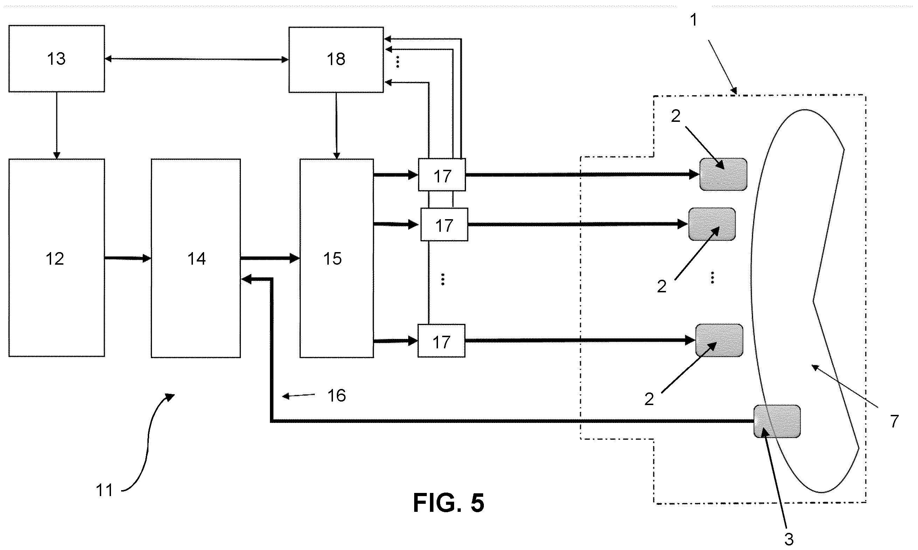

In a preferred embodiment of the invention shown in Figure 5, the controller (11) comprises a signal generator (12), preferably connected to programmable waveform-generating means (13) (typically one or more programmable integrated circuits or microcontrollers), said generator (12) being adapted to selectively conduct said waveforms to the electrodes (2) of the device. To that end, and more preferably, the controller (11) may comprise, for example, a linear power amplifier (14) of different frequencies (i.e., preferably comprised between 30 Hz and 30 kHz) and a relay block (15) for signal switching, which allow selecting the electrodes (2) of the array (1) to which the power waveform signal is sent, and those which are disconnected. The array (1) is placed around the prosthesis (7) throughout the entire process. Likewise, the reference electrodes (3) are connected to the amplifier (14) through a grounding line (16). Finally, a set of current sensors (17), for example, bipolar low-intensity sensors, provides the programmable current control means (13) with information about the current that is circulating through each of the electrodes (2) of the array (1). With this information about the current, the programmable means (13) calculate the energy supplied to each electrode (2) and deactivate same when it has reached a programmed maximum energy value. This scheme can be implemented with any number of electrodes (2). Optionally, the system can also include a user interface (18) connected to the programmable current control means (13), which allows displaying and programming the operating parameters of the system, as well as monitoring and controlling the operating condition thereof.

A third object of the invention relates to an activation method for activating a system according to any of the embodiments described above, which comprises operating the controller (11) for applying current to activate at least one of the electrodes (2) arranged in the array (1) of the device for disinfecting.

More preferably, the electrodes are activated using one or more of the following modes:
a) Sequential: in this activation mode, there is only one active electrode (2) at a time. This model simplifies the disinfection method, but increases total treatment times.
b) Parallel: in this activation mode, several or all of the electrodes (2) can be activated at the same time. To allow this mode, there is a need to use a power source which allows powering all the electrodes (2) at the same time, and this may require having one amplifier (14) and one current sensor (17) for each electrode (2). All the amplifiers (14) must be synchronized since, by using the same reference, synchronization prevents the appearance of currents between adjacent electrodes. Moreover, in order to prevent the electrodes (2) with improved resistance (due to their position with respect to the morphology of the array (1)) from applying more energy to the surface of the prosthesis (7) than the rest, they can be monitored by means of a current sensor (17). In this way, when an electrode (2) exceeds a preset current threshold value, its corresponding amplifier (14) is disconnected.
c) Mixed: in this activation mode, several sets of electrodes (2) can be activated at the same time or in sequence.

The chosen excitation signal can be a bipolar or continuous signal. The alternating signals can be sinusoidal, in the case of having linear amplifiers, or digital (for example, pulse width modulation (PWM) type), in which case work can be performed with H bridges and the electronics can be simplified considerably, increasing their efficiency and reducing their cost. The amplitude (voltage) and frequency of the alternating signals, as well as the polarity of the continuous signals, may vary. The current limit per electrode (2) is determined by the characteristics of the power source used.

All the parameters described in detail above are preferably managed by the controller (11) for applying current, the design of which allows applying different waveforms in a safe voltage interval at different frequencies for a preset time, such that the resulting currents generate the desired bioelectric effect.

### Example of a preferred embodiment of the invention:

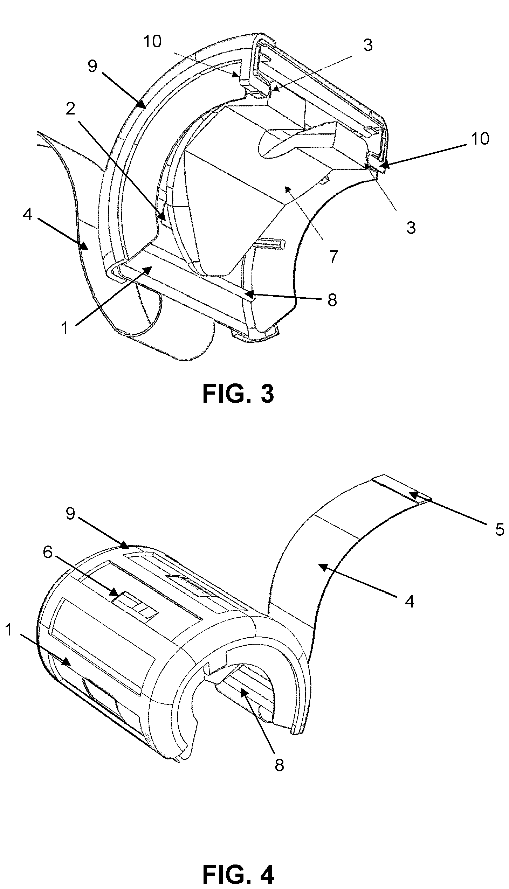

In a non-limiting example of an embodiment of the invention, the device can be made by means of an FPCB array (1) of twenty electrodes, distributed according to the pattern of Figure 1, being folded according to the pattern shown in Figures 2-4 for coupling to the femoral part of the knee prothesis (7) shown in Figure 3, using the corresponding supports (8, 9). Each electrode (2) will be monitored by a current sensor (17) in the control system (Figure 5). Signals which are transmitted to the electrodes (2) are created in a generator (12) adapted with function programmable means (13), and amplified using a linear amplifier (14) with frequencies between 30 Hz and 30 kHz (Figure 5).

In the mentioned illustrative and non-limiting example of the disinfection method which constitutes a fourth object of the invention, the array (1) of electrodes (2) is fixed to the prosthesis (7) and connected to the controller (11) by means of the terminal connector (5) of the FPCB, with an FPC-type connector (5) being used, for example. The controller (11) can be implemented using programmable means (13) adapted with known programmable integrated circuits or microcontrollers. The controller (11) may further incorporate a touch screen-based user interface (18) which allows setting the operating parameters, once the desired cycle of use has been determined, as well as displaying the progress of the system.

The controller also allows the parallel activation of different electrodes (2). Applying an alternating current of 10 Hz, with an amplitude of 4.5 V in a range between +3 and -1.5 V, gives rise to a total current, by means of parallel activation, of about 450 mA which, when applied for 20 minutes, eliminates between 90 and 99% of bacterial colonies.

From the surgical viewpoint, the disinfection method would consist of, once the patient's knee is exposed, fixing the array (1) of electrodes (2) to the prothesis (7) and bathing the area to be disinfected in saline solution. The desired current sequence in the controller (11) is then activated. The biofilm weaking time is typically comprised between 10 and 30 minutes, and more preferably between 15 and 25 minutes. Once current is applied to the prothesis, saline solution washes can be performed on the prosthesis (7), a standard antibiotic treatment for prosthetic infections.

The current application time can be reduced if the current sensors (17) detect that the desired energy has been reached before the expected time. Once said time has elapsed, the system generates a warning. The device is removed from the prosthesis (7) at that moment. The prosthesis can be washed with a sponge with, for example, povidone-iodine, and then irrigated with 5-15 liters of physiological saline solution (for a knee prosthesis (7)). Finally, the surgical wound is closed by layers.

## Claims

1. A device for intraoperatively disinfecting bone prostheses (7) using bioelectric effect, comprising an array (1) that can be adapted to the shape of a bone prosthesis (7), wherein said bone prosthesis (7) comprises one or more metal surfaces, and wherein the array (1) is provided with an arrangement of electrodes (2) adapted to be connect to a programmable controller (11) for supplying current, and adapted to perform a bioelectric treatment on the prosthesis (7),
**characterized in that** the device further comprises at least one fixing support (8) by way of a separator between the array (1) and the bone prosthesis (7), wherein said fixing support (8) is adapted to limit or prevent direct electrical contact between said bone prosthesis (7) and the electrodes (2),
and wherein the fixing support (8) is further adapted to be removed from the prosthesis after performing the bioelectric treatment.

2. The device according to the preceding claim, wherein the array (1) is made of a flexible material, on which there is arranged a plurality of conductive tracks (2') connected to a plurality of corresponding electrodes (2).

3. The device according to any of the preceding claims, wherein the array (1) comprises a central portion (1') that can be adapted to the front surface of a knee prosthesis (7), and two side portions (1") that can be adapted to the corresponding side surfaces of said prosthesis (7).

4. The device according to any of the preceding claims, wherein the array (1) comprises one or more reference electrodes (3) adapted for direct contact with the bone prosthesis (7).

5. The device according to any of the preceding claims, wherein the array (1) comprises an appendage (4), by way of a flat cable, ending in a direct-insertion connector (5), adapted to connect the device to a programmable controller (11) for supplying current.

6. The device according to any of the preceding claims, wherein the array (1) comprises one or more openings (6) adapted to favor serum circulation during the operation of the device.

7. The device according to any of the preceding claims, wherein the fixing support (8) comprises a grid, by way of a separator, between the array (1) and the prosthesis (7).

8. The device according to any of the preceding claims, comprising a structural support (9) arranged over the array (1), by way of a casing, structurally reinforcing the shape adopted by the array (1) and the fixing support (8).

9. The device according to any of the preceding claim, wherein the fixing support (8) is made with flexible materials, and the structural support (9) is made with a rigid material.

10. The device according to any of claims 8-9, wherein the structural support (9) comprises one or more holes (9') adapted to favor serum circulation during the operation of the device.

11. The device according to any of claims 8-10, wherein the structural support (9) comprises one or more positioning elements (10) adapted to position one or more reference electrodes (3) of the array (1) in contact with the bone prosthesis (7).

12. The device according to any of the preceding claims, wherein the array (1) can be sterilized and/or reused.

13. A system for intraoperatively disinfecting bone prostheses (7) using bioelectric effect, **characterized in that** the system comprises:
- a device for disinfecting according to any of the preceding claims;
- a programmable controller (11) for supplying current, connected to the device, and designed to perform a bioelectric treatment on the prosthesis (7) through the electrodes (2).

14. The system according to the preceding claim, wherein the device and the controller (11) are connected in a modular manner through a connector (5) adapted to be coupled and uncoupled thereto.

15. The system according to any of claims 13-14, wherein the controller (11) comprises a signal generator (12) connected to programmable waveform-generating means (13), said generator (12) and the programmable waveform-generating means (13) being adapted to conduct said waveforms, in a coordinated or selective manner, to the electrodes (2) of the device.

16. The system according to any of claims 13-15, wherein the controller (11) comprises one or more amplifiers (14) and a relay block (15) designed to select the electrodes (2) of the array (1) to which the power waveform signal is sent, and those which are disconnected.

17. The system according to the preceding claim, wherein one or more reference electrodes (3) are connected to the amplifiers (14) through a grounding line (16).

18. The system according to any of claims 16-17, wherein the controller (11) comprises a set of current sensors (17) connected to the relay block (15) and to the programmable current control means (13).

19. The system according to any of claims 16-18, wherein the programmable current control means (13) are designed with hardware and/or software means to calculate the energy supplied to the electrodes (2) and to deactivate them when they have reached a programmed maximum energy value.

20. The system according to any of claims 16-19, wherein the controller (11) comprises a user interface (18) connected to the programmable current control means (13) designed to display and program the operating parameters of the system, as well as to monitor and control the operating condition thereof.

21. An activation method for activating the system according to any of claims 13-20, which comprises operating the controller (11) for applying current to activate at least one of the electrodes (2) arranged in the array (1) of the device for disinfecting.

22. The method according to the preceding claim, wherein the electrodes (2) are activated by means of at least one of the following modes:
- sequential, wherein there is only one active electrode (2) at a time;
- parallel, wherein several or all of the electrodes (2) are activated at the same time.
